(19) Europäisches Patentamt
European Patent Office
Office européen des brevets

(11) Veröffentlichungsnummer: **0 371 342 B1**

(12) **EUROPÄISCHE PATENTSCHRIFT**

(45) Veröffentlichungstag der Patentschrift: **14.12.94**

(51) Int. Cl.5: **C07D 209/30**, A61K 31/405

(21) Anmeldenummer: **89121251.6**

(22) Anmeldetag: **17.11.89**

Die Akte enthält technische Angaben, die nach dem Eingang der Anmeldung eingereicht wurden und die nicht in dieser Patentschrift enthalten sind.

# (54) 2-Halogensubstituierte N-Indolylethyl-sulfonsäureamide, Verfahren zu ihrer Herstellung und ihre Verwendung in Arzneimitteln.

(30) Priorität: **30.11.88 DE 3840338**

(43) Veröffentlichungstag der Anmeldung:
**06.06.90 Patentblatt 90/23**

(45) Bekanntmachung des Hinweises auf die Patenterteilung:
**14.12.94 Patentblatt 94/50**

(84) Benannte Vertragsstaaten:
**AT BE CH DE ES FR GB GR IT LI LU NL SE**

(56) Entgegenhaltungen:
**DE-A- 3 514 696**
**DE-A- 3 613 623**

(73) Patentinhaber: **BAYER AG**

**D-51368 Leverkusen (DE)**

(72) Erfinder: **Böshagen, Horst, Dr.**
**Wiesenstrasse 4**
**D-5657 Haan (DE)**
Erfinder: **Perzborn, Elizabeth, Dr.**
**Am Tescher Busch 13**
**D-5600 Wuppertal 11 (DE)**
Erfinder: **Fiedler, Volker-Bernd, Dr.**
**Lehner Mühle 46**
**D-5090 Leverkusen 3 (DE)**

**EP 0 371 342 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 2-halogensubstituierte N-Indolylethyl-sulfonsäureamide, Verfahren zu ihrer Herstellung und ihre Verwendung in Arzneimitteln.

Es ist bekannt, daß N-Indolylethyl-sulfonamide und N-Dihydroindolylethyl-sulfonamide eine thrombozytenaggregationshemmende und Thromboxan $A_2$-antagonistische Wirkung besitzen [vgl. DOS 35.14.696 und DOS 36.13.623].

Es wurden nun neue 2-halogenierte N-Indolylethyl-sulfonsäureamide der allgemeinen Formel (I)

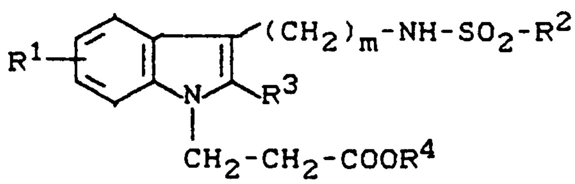

(I)

in welcher

$R^1$
- für Wasserstoff, Halogen, Trifluormethyl, Carboxy, Alkoxycarbonyl mit bis zu 8 Kohlenstoffatomen steht, oder
- für geradkettiges oder verzweigtes Alkyl mit bis zu 8 Kohlenstoffatomen oder für Cycloalkyl mit 3 bis 8 Kohlenstoffatomen steht, das gegebenenfalls durch Carboxy, Halogen, Alkoxy oder Alkoxycarbonyl mit jeweils bis zu 8 Kohlenstoffatomen, Cyano oder Hydroxy substituiert ist, oder
- für eine Gruppe der Formel $-S(O)_n-R^5$, $-NR^6R^7$ oder $-OR^8$ steht

worin

$R^5$
- geradkettiges oder verzweigtes Alkyl mit bis zu 8 Kohlenstoffatomen oder Aryl mit 6 bis 10 Kohlenstoffatomen bedeutet

$R^6$ und $R^7$ gleich oder verschieden sind und Wasserstoff, geradkettiges oder verzweigtes Alkyl mit bis zu 8 Kohlenstoffatomen, Aryl mit 6 bis 10 Kohlenstoffatomen oder Acetyl bedeuten,

$R^8$
- Wasserstoff, geradkettiges oder verzweigtes Alkyl oder Alkylsulfonyl mit bis zu 8 Kohlenstoffatomen, Aryl oder Arylsulfonyl mit jeweils 6 bis 10 Kohlenstoffatomen oder Trifluormethyl bedeutet,

und

n
- eine Zahl 0, 1 oder 2 bedeutet

$R^2$
- für geradkettiges oder verzweigtes Alkyl mit bis zu 10 Kohlenstoffatomen steht, das gegebenenfalls durch Halogen, Phenyl, Hydroxy oder Cyano substituiert ist, oder
- für Aryl mit 6 bis 10 Kohlenstoffatomen steht, das gegebenenfalls bis zu 5-fach gleich oder verschieden durch Halogen, Cyano, Trifluormethyl, Trifluormethoxy, Trifluormethylthio oder durch Alkyl, Alkoxy, Alkoxycarbonyl oder Alkylthio mit jeweils bis zu 8 Kohlenstoffatomen, Hydroxy, Carboxy, Phenyl, Phenoxy, Benzyloxy, Benzylthio oder durch eine Gruppe der Formel $-NR^6R^7$ substituiert ist,

worin

$R^6$ und $R^7$ die oben angegebene Bedeutung haben,

$R^3$
- für Halogen steht,

$R^4$
- für Wasserstoff, geradkettiges oder verzweigtes Alkyl mit bis zu 8 Kohlenstoffatomen oder für Phenyl oder Amino steht

und

m
- für eine der Zahlen 2, 3 oder 4 steht,

und deren Salze gefunden.

Die erfindungsgemäßen 2-halogensubstituierten N-Indolylethyl-sulfonsäureamide können auch in Form ihrer Salze vorliegen. Im allgemeinen seien hier Salze mit organischen oder anorganischen Basen genannt.

Im Rahmen der vorliegenden Erfindung werden physiologisch unbedenkliche Salze bevorzugt. Physiologisch unbedenkliche Salze der 2-halogensubstituierten-N-Indolylethyl-sulfonsäureamide können Metall- oder Ammoniumsalze der erfindungsgemäßen Stoffe, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin oder Ethylendiamin.

Die erfindungsgemäßen Stoffe zeigen überraschenderweise eine thrombozytenaggregationshemmende und eine Thromboxan A2-antagonistische Wirkung und können zur therapeutischen Behandlung von Menschen und Tieren verwendet werden.

Bevorzugt werden Verbindungen der allgemeinen Formel (I),
in welcher

$R^1$
- für Wasserstoff, Fluor, Chlor, Brom, Trifluormethyl, Carboxy, Alkoxycarbonyl mit bis zu 6 Kohlenstoffatomen steht, oder
- für geradkettiges oder verzweigtes Alkyl mit bis zu 6 Kohlenstoffatomen oder für Cycloalkyl mit 3 bis 6 Kohlenstoffatomen steht, das gegebenenfalls durch Carboxy, Fluor, Chlor, Brom, Alkoxy oder Alkoxycarbonyl mit jeweils bis zu 6 Kohlenstoffatomen, Cyano oder Hydroxy substituiert ist, oder
- für eine Gruppe der Formel $-S(O)_n-R^5$, $-NR^6R^7$ oder $-OR^8$ steht,

worin

$R^5$
- geradkettiges oder verzweigtes Alkyl mit bis zu 6 Kohlenstoffatomen oder Phenyl bedeutet,

$R^6$ und $R^7$ gleich oder verschieden sind und Wasserstoff, geradkettiges oder verzweigtes Alkyl mit bis zu 6 Kohlenstoffatomen, Phenyl oder Acetyl bedeuten,

$R^8$
- Wasserstoff, geradkettiges oder vezweigtes Alkyl oder Alkylsulfonyl mit jeweils bis zu 6 Kohlenstoffatomen, Phenyl oder Trifluormethyl bedeutet,

und

n
- eine Zahl 0, 1 oder 2 bedeutet,

$R^2$
- für geradkettiges oder verzweigtes Alkyl mit bis zu 8 Kohlenstoffatomen steht, das gegebenenfalls durch Fluor, Chlor, Brom, Phenyl, Hydroxy oder Cyano substituiert ist, oder
- für Phenyl steht, das gegebenenfalls bis zu 3-fach gleich oder verschieden durch Fluor, Chlor, Brom, Cyano, Trifluormethyl, Trifluormethoxy, Trifluormethylthio oder durch Alkyl, Alkoxy, Alkoxycarbonyl oder Alkylthio mit jeweils bis zu 6 Kohlenstoffatomen, Hydroxy, Carboxy, Phenyl, Phenoxy, Benzyloxy, Benzylthio oder durch eine Gruppe der Formel $-NR^6R^7$ substiutiert ist,

worin
$R^6$ und $R^7$ die oben angegebene Bedeutung haben,

$R^3$
- für Fluor, Chlor, Brom oder Iod steht,

$R^4$
- für Wasserstoff, geradkettiges oder verzweigtes Alkyl mit bis zu 6 Kohlenstoffatomen oder für Phenyl steht

und

m
- für eine der Zahlen 2 oder 3 steht,

und deren Salze.

Besonders bevorzugt werden Verbindungen der allgemeinen Formel (I),
in welcher

$R^1$
- für Wasserstoff, Fluor, Chlor oder Trifluormethyl, steht, oder
- für Methyl, Ethyl oder Propyl steht, oder

- für eine Gruppe der Formel $-NR^6R^7$ oder $-OR^8$ steht,

worin

$R^6$ und $R^7$ gleich oder verschieden sind und Wasserstoff, Methyl, Ethyl oder Propyl, bedeuten,

und

$R^8$

- Wasserstoff, Methyl oder Ethyl bedeutet,

$R^2$

- für Methyl, Ethyl, Propyl, Isopropyl, Butyl oder tert.Butyl steht, das gegebenenfalls durch Fluor, Chlor, Phenyl, Hydroxy oder Cyano substituiert ist, oder
- für Phenyl steht, das gegebenenfalls bis zu 3-fach gleich oder verschieden durch Fluor, Chlor, Brom, Cyano, Trifluormethyl, Trifluormethoxy, Trifluormethylthio oder durch Methyl, Ethyl, Propyl, Isopropyl, Butyl, tert.Butyl, Methoxy, Ethoxy, Methoxycarbonyl, Ethoxycarbonyl, Methylthio, Ethylthio, Hydroxy, Carboxy, Phenyl, Phenoxy, Benzyloxy, Benzylthio oder durch eine Gruppe der Formel $-NR^6R^7$ substituiert ist,

worin

$R^6$ und $R^7$ die oben angegebene Bedeutung haben,

$R^3$

- für Fluor, Chlor oder Brom steht,

$R^4$

- für Wasserstoff, geradkettiges oder verzweigtes Alkyl mit bis zu 4 Kohlenstoffatomen oder Phenyl steht,

m

- für die Zahl 2 steht,

und deren Salze.

Weiterhin wurde ein Verfahren zur Herstellung der erfindungsgemäßen Verbindungen der allgemeinen Formel (I) gefunden, das dadurch gekennzeichnet ist, daß man Indole der allgemeinen Formel (II)

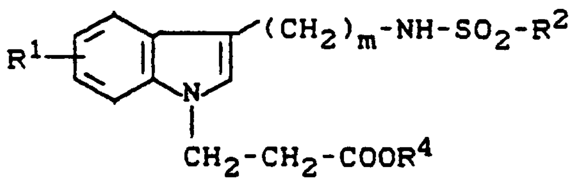

(II)

in welcher

$R^1$, $R^2$, $R^4$ und m die oben angegebene Bedeutung haben,

mit Halogen wie Fluor, Chlor oder Brom umsetzt,

und im Fall, daß $R^4$ für Alkyl oder Phenyl steht, mit Basen zu den entsprechenden Säuren ($R^4$ = Wasserstoff) verseift und im Fall, daß $R^4$ für Amino steht, die entsprechenden Ester oder Säuren ($R^4$ = Wasserstoff, Alkyl oder Phenyl) mit Ammoniak unter üblichen aktivierten Bedingungen behandelt.

Bevorzugt ist die Umsetzung der Indole der allgemeinen Formel (II), in welcher $R^4$ für Alkyl steht.

Die Indole der allgemeinen Formel (II) sind größtenteils bekannt [vgl. DOS 35.14.696] oder können nach der in DOS 36.13.623 beschriebenen Methode dargestellt werden.

Die erfindungsgemäßen Verfahrensstufen können durch die folgenden Formelschemata erläutert werden:

I.

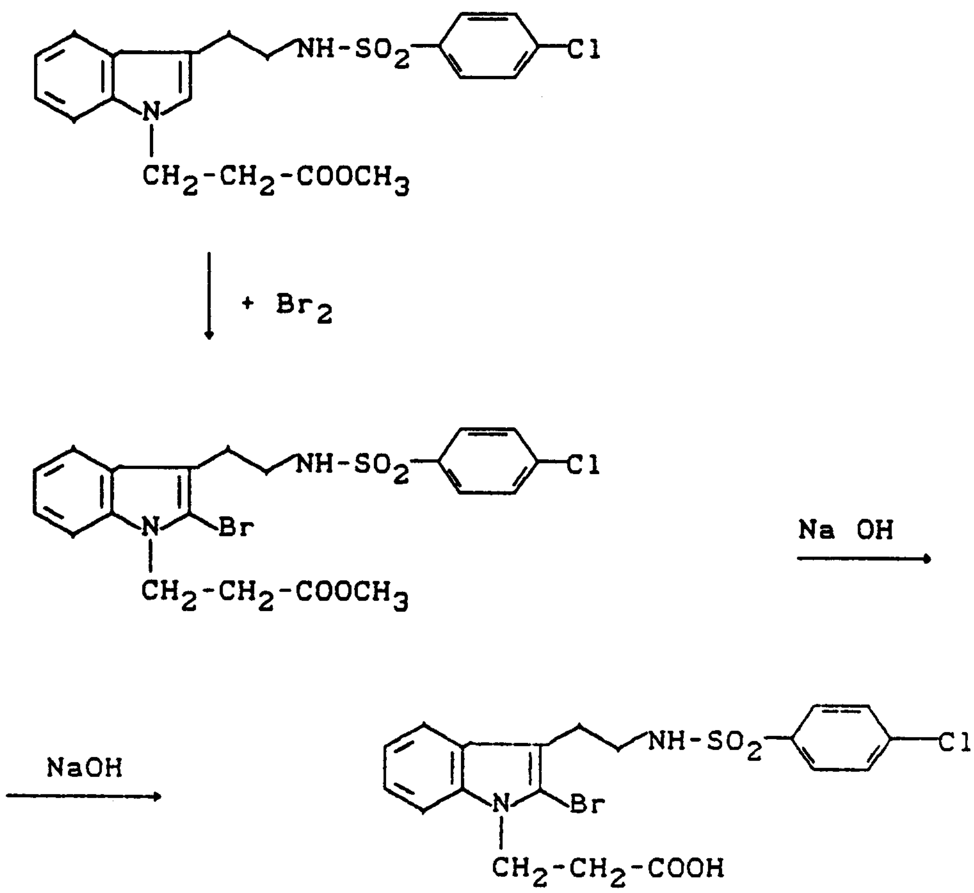


II.

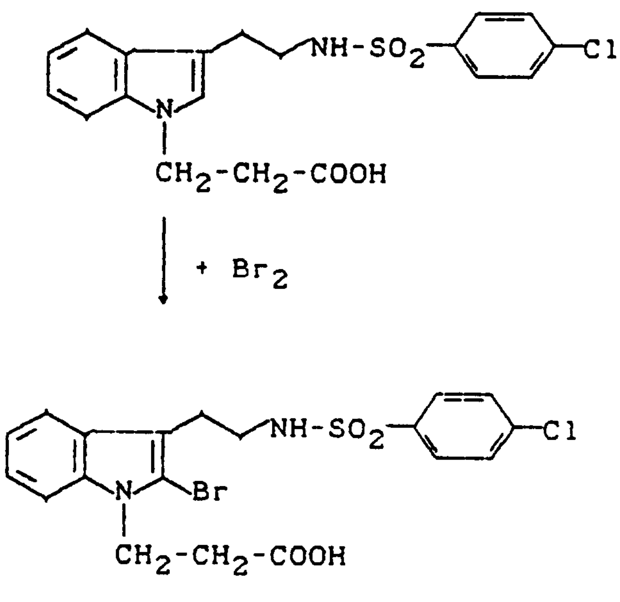


Lösemittel für die erfindungsgemäßen Verfahrensstufen können inerte organische Lösemittel sein, die sich unter den Reaktionsbedingungen nicht verändern. Hierzu gehören bevorzugt Kohlenwasserstoffe wie Benzol, Toluol, Xylol, Cyclohexan, Hexan oder Erdölfraktionen, Chlorkohlenwasserstoffe wie Methylenchlorid, Chloroform, Dimethylformamid oder Dimethylsulfoxid.

Die Halogenierung wird im allgemeinen in einem Temperaturbereich von -20 °C bis +30 °C, bevorzugt von -10 °C bis 0 °C durchgeführt.

Im allgemeinen setzt man 1 bis 20 Mol, bevorzugt 1 bis 10 Mol Halogen bezogen auf 1 Mol der Indole der allgemeinen Formel (II) ein.

Die erfindungsgemäßen Verfahrensstufen werden im allgemeinen bei Normaldruck durchgeführt. Es ist aber auch möglich, die Verfahren bei Unterdruck oder Überdruck durchzuführen (z.B. in einem Druckbereich von 0,5 bis 5 bar).

Die Verseifung der Verbindungen der allgemeinen Formel (I) in welcher $R^4$ für Alkyl oder Phenyl steht, zu den entsprechenden Säuren, erfolgt in an sich bekannter Weise in Gegenwart von Basen, wie Alkali- oder Erdalkalihydroxiden oder -alkanolaten, in Lösemitteln wie Wasser oder Alkoholen. Bevorzugt werden als Basen Natrium-, Kalium- oder Bariumhydroxid, Natriummethanolat, Kaliummethanolat, Natriumethanolat oder Kaliumethanolat, bevorzugt in Wasser oder Methanol, Ethanol, Propanol, oder Isopropanol, oder in Gemischen dieser Lösemittel eingesetzt.

Im allgemeinen setzt man 1 bis 100 Mol, bevorzugt 2 bis 20 Mol Base, bezogen auf 1 Mol der 2-halogensubstituierten-N-{2-[1-(2-Alkoxycarbonylethyl)-1H-indol-3-yl]}ethylsulfonamide ein.

Die Verseifung wird in einem Temperaturbereich von 0°C bis 100°C, bevorzugt von 20°C bis 80°C durchgeführt.

Die neuen 2-halogensubstituierten N-Indolylethyl-sulfonsäureamide bzw. deren Salze können als Wirkstoffe in Arzneimitteln eingesetzt werden. Die Wirkstoffe weisen eine thrombozytenaggregationshemmende und Thromboxan $A_2$-antagonistische Wirkung auf. Sie können bevorzugt zur Behandlung von Thrombosen, Thromboembolien, Ischämien, als Antiasthmatika und als Antiallergika eingesetzt werden.

Zur Bestimmung der thrombozytenaggregationshemmenden Wirkung wurde Blut von gesunden Probanden beiderlei Geschlechts verwendet. Als Antikoagulans wurden einem Teil 3,8%iger wäßriger Natriumzitratlösung 9 Teile Blut zugemischt. Mittels Zentrifugation erhält man aus diesem Blut plättchenreiches Zitratplasma (PRP) (Jürgens/Beller, Klinische Methoden der Blutgerinnungsanalyse; Thieme Verlag, Stuttgart, 1959).

Für diese Untersuchungen wurden 0,8 ml PRP und 0,1 ml der Wirkstofflösung bei 37°C im Wasserbad vorinkubiert. Anschließend wurde die Thrombozytenaggregation nach der turbidometrischen Methode (Born, G.V.R., J. Physiol. (London), 162, 67, 1962) im Aggregometer bei 37°C bestimmt (Therapeutische Berichte 47, 80-86, 1975). Hierzu wurde die vorinkubierte Probe mit 0,1 ml Kollagen, einem aggregationsauslösenden Agens, versetzt. Die Veränderung der optischen Dichte in der Probe der PRP wurde während einer Zeitdauer von 6 Minuten aufgezeichnet und der Ausschlag nach 6 Minuten bestimmt. Hierzu wird die prozentuale Hemmung gegenüber der Kontrolle errechnet. Als Grenzkonzentration wird der Bereich der minimal effektiven Konzentration angegeben.

Die Grenzkonzentrationen liegen zwischen 0,003 - 10 mg/l.

Beispiel 21:
EC = 0,01 - 0,03 mg/l.
Beispiel 22:
EC = 0,03 - 0,1 mg/l.

Die neuen Wirkstoffe können in an sich bekannter Weise unter Verwendung inerter nichttoxischer, pharmazeutisch geeigneter Trägerstoffe oder Lösungsmittel in die üblichen Formulierungen überführt werden, wie Tabletten, Kapseln, Dragees, Pillen, Granulate, Aerosole, Sirupe, Emulsionen, Suspensionen und Lösungen. Hierbei soll die therapeutisch wirksame Verbindung jeweils in einer Konzentration von etwa 0,5 bis 90 Gew.-%, bevorzugt von 5 bis 70 Gew.-% vorliegen, die ausreichend ist, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nichttoxische organische Lösungsmittel wie Paraffine (z. B. Erdölfraktionen), pflanzliche Öle (z. B. Erdnuß/Sesamöl), Alkohole (z.B. Ethylalhohol, Glycerin), Glykole (z.B. Propylenglykol, Propylethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäureester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation kann in üblicher Weise erfolgen, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen. Bei oraler Applikation beträgt die Dosierung im allgemeinen etwa 0,01 bis 20 mg/kg, vorzugsweise 0,1 bis 10 mg/kg Körpergewicht.

Es kann aber gegebenenfalls vorteilhaft sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehrere Einzelgaben über den Tag zu verteilen.

Beispiel 1

N-[2-(1H-Indol-3-yl)]ethyl-(4-methylphenyl)sulfonamid

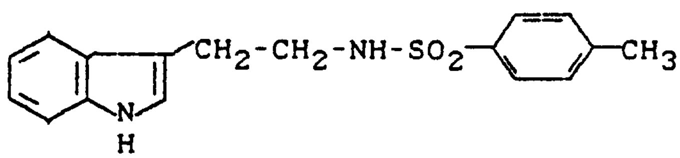

17,5 g (110 mmol) Tryptamin und 18,0 g (220 mmol) Natriumacetat werden in 250 ml Ethanol gelöst. Bei 0 °C werden 20,97 g (110 mmol) p-Toluolsulfonsäurechlorid in 100 ml Ethanol gelöst zugetropft. Man rührt erst 1 h bei Raumtemperatur, dann 1 h bei Rückfluß. Anschließend gibt man so viel Wasser zu, bis eine klare Lösung entsteht. Ein Teil des Ethanols wird abdestilliert. Das Produkt kristallisiert aus, wird abgesaugt und aus Isopropanol umkristallisiert.

Ausbeute: 20.7 g (60% der Theorie)
Schmp.: 118 °C
$R_f$ = 0,6 (Toluol:Ethanol 3:1)
DC-Alufolie Kieselgel 60 $F_{254}$, Schichtdicke 0,2 mm, Art. 5562 Merck

Beispiel 2

N-[2-Cyanoethyl]-N-{2-[1-(2-cyanoethyl)-1H-indol-3-yl]-ethyl-(4-methylphenyl)sulfonamid

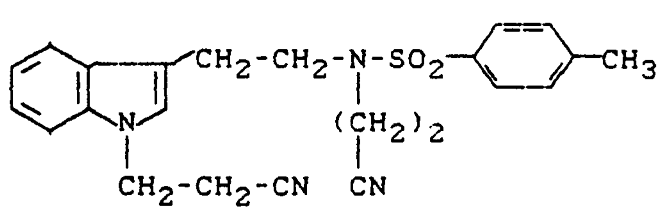

10 g der Verbindung aus Beispiel 1 werden in 150 ml Dioxan gelöst. Dann werden 10ml Acrylnitril zugegeben. Zu dieser Lösung gibt man 1 ml Benzyltrimethylammoniumhydroxidlösung (40%ig) gelöst in 4 ml Methanol. Es wird 5 h bei 80 °C gerührt und dann in Wasser gegeben. Es wird dreimal mit Essigester extrahiert. Man wäscht die organische Phase mit Wasser, trocknet über $MgSO_4$, saugt ab und engt am Rotationsverdampfer ein. Mit wenig Ether kristallisiert das Produkt aus. Es wird aus Ethanol/Acetonitril (1:1) umkristallisiert.

Ausbeute: 10,1 g (75,5% der Theorie)
Schmp.: 88 °C
$R_f$ = 0,5 (Toluol:Ethanol 3:1)
DC-Alufolie Kieselgel 60 $F_{254}$, Schichtdicke 0,2 mm, Art. 5562 Merck

Beispiel 3

N-{2-[1-(2-Carboxyethyl)-1H-indol-3-yl]}ethyl-(4-methylphenyl)sulfonamid

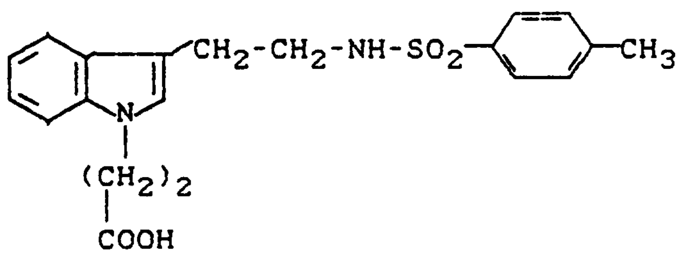

10 g der Verbindung aus Beispiel 2 werden in 300 ml 10%iger Kalilauge 3 h bei Rückfluß gerührt. Dann stellt man mit 6 molarer HCl sauer und extrahiert dreimal mit Chloroform. Die organische Phase wird zweimal mit Wasser gewaschen, über $MgSO_4$ getrocknet und am Rotationsverdampfer eingeengt. Mit wenig Essigester kristallisiert das Produkt aus und wird abgesaugt.
Ausbeute: 4,7 g (51,1% der Theorie)
Schmp.: 126 °C
$R_f$ = 0,32 (Toluol:Ethanol 3:1)
DC-Alufolie Kieselgel 60 $F_{254}$, Schichtdicke 0,2 mm, Art. 5562 Merck

Beispiel 4

N-{2-[1-(2-Methoxycarbonyl-ethyl)-1H-indol-3-yl]}ethyl-(4-methyl-phenyl)sulfonamid

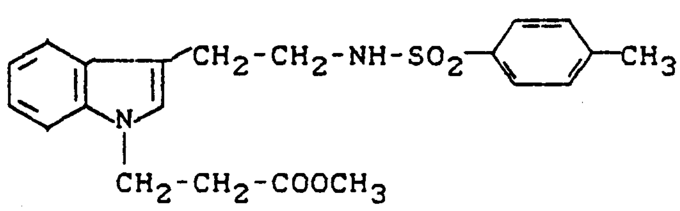

400 ml Methanol werden unter Rühren bei -20 °C tropfenweise mit 100 g Thionylchlorid versetzt. Nach beendeter Zugabe wird noch 5 Minuten bei gleicher Temperatur gerührt. Es wird auf -50 °C gekühlt und 20 g (54 mmol) der Verbindung aus Beispiel 3 werden zugegeben. Nach 2-stündigem Rühren bei Raumtemperatur wird die Reaktionsmischung am Rotationsverdampfer eingeengt. Der verbleibende Rückstand wird in Essigester gelöst und nacheinander mit gesättigter Natriumhydrogencarbonatlösung und Wasser gewaschen, über Magnesiumsulfat getrocknet und eingeengt. Mit Ether kristallisiert das Produkt aus und wird abgesaugt.
Ausbeute: 20,0 g (96,5% der Theorie)
Schmp.: 101 °C
$R_f$ = 0,63 (Toluol:Ethanol 3:1)
DC-Alufolie Kieselgel 60 $F_{254}$, Schichtdicke 0,2 mm, Art. 5562 Merck
Die in der Tabelle 1 aufgeführten Verbindungen wurden analog der Vorschrift von Beispiel 4 hergestellt:

Tabelle 1:

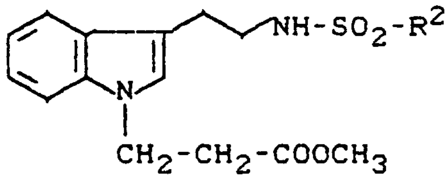

| Beispiel-Nr. | $R^2$ | Fp.: | $R_f$.:* |
|---|---|---|---|
| 5 | 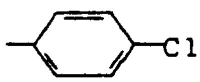 | 93° C | 0,68 |
| 6 | 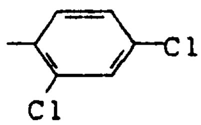 | 105° C | 0,71 |
| 7 | 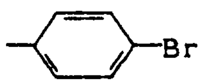 | 87° C | 0,69 |
| 8 | 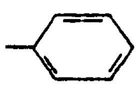 | 96° C | 0,67 |

* (Toluol/Ethanol 3:1)
DC-Alufolie Kieselgel 60 $F_{254}$, Schichtdicke 0,2 mm, Art. 5562 Merck

Beispiel 9

2-Brom-3-[2-(4-tolylsulfonylamino)ethyl]-indol-1-propionsäuremethylester

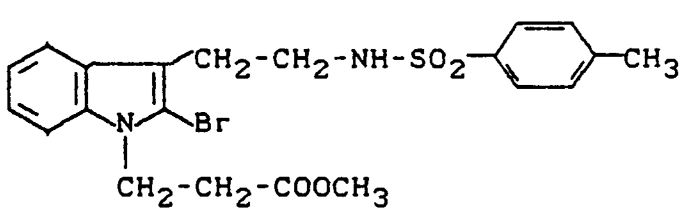

4,0 g (10 mmol) der Verbindung aus Beispiel 4 werden in 250 ml absolutem Methylenchlorid gelöst. Bei -5 ° C werden innerhalb von 45 Minuten 1,6 g Brom in 200 ml absolutem Methylenchlorid zugetropft. Nach beendeter Zugabe wird noch 30 Minuten bei 0 ° C nachgerührt. Das Reaktionsgemisch wird 2 mal mit gesättigter Natriumhydrogencarbonatlösung gewaschen, über Magnesiumsulfat getrocknet und am Rotationsverdampfer eingeengt. Durch Chromatographie an Kieselgel und Toluol:Essigester (2:1) als Elutionsmittel erhält man ein hellgelbes Harz.
Ausbeute: 3,5 g (73,1% der Theorie)
$R_f$ = 0,69 (Toluol:Essigester 3:1)
DC-Alufolie Kieselgel 60 $F_{254}$, Schichtdicke 0,2 mm, Art. 5562 Merck

Die in der Tabelle 2 aufgeführten Verbindungen wurden analog der Vorschrift von Beispiel 9 hergestellt:

Tabelle 2:

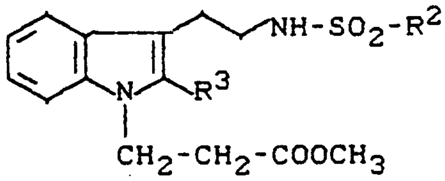

| Beisp.-Nr.: | $R^2$ | $R^3$ | Fp.: | $R_f$.:* |
|---|---|---|---|---|
| 10 | 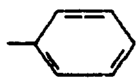 | Br | 93° C | 0,70 |
| 11 | 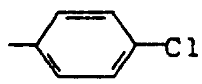 | Br | 112° C | 0,61 |

Fortsetzung Tabelle 2:

| Beisp.-Nr.: | $R^2$ | $R^3$ | Fp.: | $R_f$.:* |
|---|---|---|---|---|
| 12 | 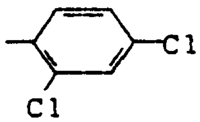 | Br | - | 0,72 |
| 13 | 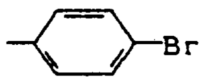 | Br | 122° C | 0,68 |
| 14 | 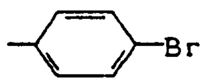 | Cl | 113° C | 0,68 |
| 15 | 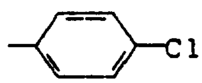 | Cl | 89° C | 0,71 |
| 16 | 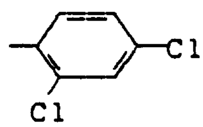 | Cl | - | 0,72 |

* (Toluol/Ethanol 3:1)
DC-Alufolie Kieselgel 60 $F_{254}$, Schichtdicke 0,2 mm, Art. 5562 Merck

Beispiel 17

2-Brom-3-[2-(4-toluylsulfonylamino)ethyl]-indol-1-propionsäure

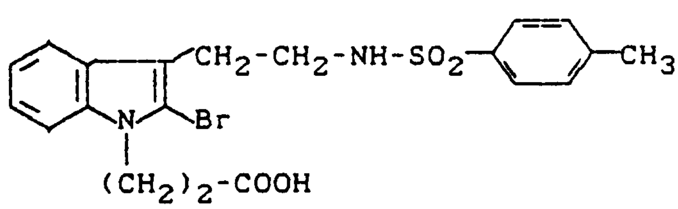

Eine Lösung von 2,7 g (5,6 mmol) der Verbindung aus Beispiel 9 in 60 ml 1,2-Dimethoxyethan wird bei 0 °C mit 20 ml 1 molarer Natronlauge versetzt und 30 Minuten bei 0 °C, dann 1 Stunde bei Raumtemperatur gerührt. Die wässrige Phase wird abgetrennt und 2 mal mit Ether extrahiert. Anschließend wird die Wasserphase unter Eiskühlung mit 1 molarer Salzsäure sauer gestellt. Das ausgefallene ölige Produkt wird 3 mal mit Methylenchlorid extrahiert, die vereinigten organischen Phasen werden mit Wasser gewaschen, über Magnesiumsulfat getrocknet und am Rotationsverdampfer eingeengt. Mit wenig Toluol kristallisiert das Produkt und wird abgesaugt.
Ausbeute: 2,0 g (76,3% der Theorie)

Schmp.;: 118 ° C
$R_f$ = 0,50 (Toluol:Ethanol 3:1)
DC-Alufolie Kieselgel 60 $F_{254}$, Schichtdicke 0,2 mm, Art. 5562 Merck

Beispiel 18

2-Brom-3-[2-(4-fluoro-phenylsulfonylamino)ethyl]-indol-1-propionsäure

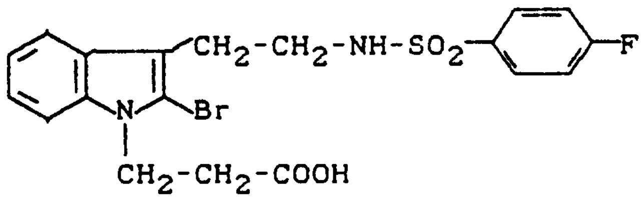

1,45 g (5 mmol) N-{2-[1-(2-carboxyethyl)-1H-indol-3-yl]}ethyl-(4-fluor-phenyl)-sulfonamid werden in 100 ml absolutem Methylenchlorid gelöst und bei 0 ° C tropfenweise innerhalb 1 Stunde mit 0,26 ml Brom in 100 ml Methylenchlorid versetzt. Das Reaktionsgemisch wird eine weitere Stunde bei 0 ° C gerührt, danach am Rotationsverdampfer eingeengt. Durch Chromatographie an Kieselgel und Toluol/Ethanol (6:1) als Elutionsmittel erhält man ein hellgelbes Öl, welches man durch ein Methylenchlorid/Ether-Gemisch zur Kristallisation bringt.
Ausbeute: 303 mg (17,5% der Theorie)
Schmp.: 131 ° C
$R_f$ = 0,46
DC-Alufolie Kieselgel 60 $F_{254}$, Schichtdicke 0,2 mm, Art. 5562 Merck
Die in der Tabelle 3 aufgeführten Verbindungen wurden analog den Vorschriften der Beispiele 17 und 18 hergestellt.

Tabelle 3:

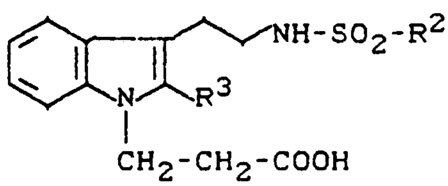

| Beisp.-Nr.: | $R^2$ | $R^3$ | Fp.: | $R_f$.:* |
|---|---|---|---|---|
| 19 | 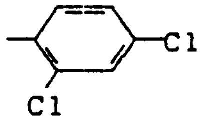 | Br | 142° C | 0,43 |

Fortsetzung Tabelle 3:

| Beisp.-Nr.: | $R^2$ | $R^3$ | Fp.: | $R_f$.:* |
|---|---|---|---|---|
| 20 | 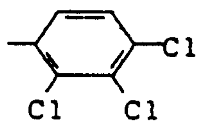 | Br | 169° C | 0,37 |
| 21 | 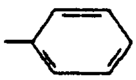 | Br | 115° C | 0,54 |
| 22 | 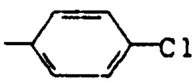 | Br | 126° C | 0,40 |
| 23 | 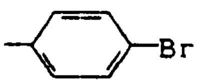 | Br | 125° C | 0,50 |
| 24 | 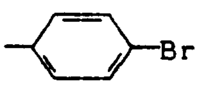 | Cl | 136° C | 0,47 |
| 25 | 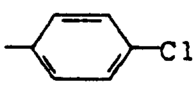 | Cl | 165° C | 0,53 |
| 26 | 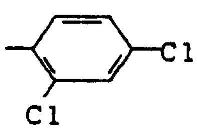 | Cl | 160° C | 0,55 |

* (Toluol/Ethanol 3:1)
DC-Alufolie Kieselgel 60 $F_{254}$, Schichtdicke 0,2 mm, Art. 5562 Merck

## Patentansprüche

1. 2-Halogensubstituierte N-Inolylethyl-sulfonamide der allgemeinen Formel (I)

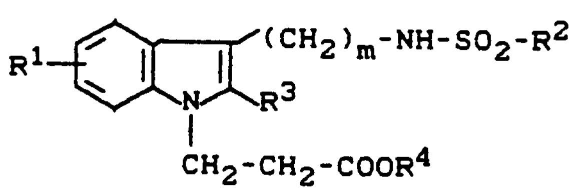

(I)

in welcher

$R^1$

- für Wasserstoff, Halogen, Trifluormethyl, Carboxy, Alkoxycarbonyl mit bis zu 8 Kohlenstoffatomen steht, oder
- für geradkettiges oder verzweigtes Alkyl mit bis zu 8 Kohlenstoffatomen oder für Cycloalkyl mit 3 bis 8 Kohlenstoffatomen steht, das gegebenenfalls durch Carboxy, Halogen, Alkoxy oder Alkoxycarbonyl mit jeweils bis zu 8 Kohlenstoffatomen, Cyano oder Hydroxy substituiert ist, oder
- für eine Gruppe der Formel $-S(O)_nR^5$, $-NR^6R^7$ oder $-OR^8$ steht,

worin

$R^5$

- geradkettiges oder verzweigtes Alkyl mit bis zu 8 Kohlenstoffatomen oder Aryl mit 6 bis 10 Kohlenstoffatomen bedeutet,

$R^6$ und $R^7$ gleich oder verschieden sind und Wasserstoff, geradkettiges oder verzweigtes Alkyl mit bis zu 8 Kohlenstoffatomen, Aryl mit 6 bis 10 Kohlenstoffatomen oder Acetyl bedeuten,

$R^8$

- Wasserstoff, geradkettiges oder verzweigtes Alkyl oder Alkylsulfonyl mit bis zu 8 Kohlenstoffatomen, Aryl oder Arylsulfonyl mit jeweils 6 bis 10 Kohlenstoffatomen oder Trifluormethyl bedeutet,

und

n

- eine Zahl 0, 1 oder 2 bedeutet

$R^2$

- für geradkettiges oder verzweigtes Alkyl mit bis zu 10 Kohlenstoffatomen steht, das gegebenenfalls durch Halogen, Phenyl, Hydroxy oder Cyano substituiert ist, oder
- für Aryl mit 6 bis 10 Kohlenstoffatomen steht, das gegebenenfalls bis zu 5-fach gleich oder verschieden durch Halogen, Cyano, Trifluormethyl, Trifluormethoxy, Trifluormethylthio oder durch Alkyl, Alkoxy, Alkoxycarbonyl oder Alkylthio mit jeweils bis zu 8 Kohlenstoffatomen, Hydroxy, Carboxy, Phenyl, Phenoxy, Benzyloxy, Benzylthio oder durch eine Gruppe der Formel $-NR^6R^7$ substituiert ist,

worin

$R^6$ und $R^7$ die oben angegebene Bedeutung haben,

$R^3$

- für Halogen steht,

$R^4$

- für Wasserstoff, geradkettiges oder verzweigtes Alkyl mit bis zu 8 Kohlenstoffatomen oder für Phenyl steht

und

m

- für eine der Zahlen 2, 3 oder 4 steht, der Salze.

**2.** 2-Halogensubstituierte N-Indolylethyl-sulfonamide der Formel (I) nach Anspruch 1 worin

$R^1$

- für Wasserstoff, Fluor, Chlor, Brom, Trifluormethyl, Carboxy, Alkoxycarbonyl mit bis zu 6 Kohlenstoffatomen steht, oder
- für geradkettiges oder verzweigtes Alkyl mit bis zu 6 Kohlenstoffatomen oder für Cycloalkyl mit 3 bis 6 Kohlenstoffatomen steht, das gegebenenfalls durch Carboxy, Fluor, Chlor, Brom, Alkoxy oder Alkoxycarbonyl mit jeweils bis zu 6 Kohlenstoffatomen, Cyano oder Hydroxy substituiert ist, oder
- für eine Gruppe der Formel $-S(O)_n-R^5$, $-NR^6R^7$ oder $-OR^8$ steht,

worin

$R^5$

- geradkettiges oder verzweigtes Alkyl mit bis zu 6 Kohlenstoffatomen oder Phenyl bedeutet,

$R^6$ und $R^7$ gleich oder verschieden sind und Wasserstoff, geradkettiges oder verzweigtes Alkyl mit bis zu 6 Kohlenstoffatomen, Phenyl oder Acetyl bedeuten,

$R^8$
- Wasserstoff, geradkettiges oder verzweigtes Alkyl oder Alkylsulfonyl mit jeweils bis zu 6 Kohlenstoffatomen, Phenyl oder Trifluormethyl bedeutet,

und

n
- eine Zahl 0, 1 oder 2 bedeutet,

$R^2$
- für geradkettiges oder verzweigtes Alkyl mit bis zu 8 Kohlenstoffatomen steht, das gegebenenfalls durch Fluor, Chlor, Brom, Phenyl, Hydroxy oder Cyano substituiert ist, oder
- für Phenyl steht, das gegebenenfalls bis zu 3-fach gleich oder verschieden durch Fluor, Chlor, Brom, Cyano, Trifluormethyl, Trifluormethoxy, Trifluormethylthio oder durch Alkyl, Alkoxy, Alkoxycarbonyl oder Alkylthio mit jeweils bis zu 6 Kohlenstoffatomen, Hydroxy, Carboxy, Phenyl, Phenoxy, Benzyloxy, Benzylthio oder durch eine Gruppe der Formel $-NR^6R^7$ substiutiert ist,

worin
$R^6$ und $R^7$ die oben angegebene Bedeutung haben,

$R^3$
- für Fluor, Chlor, Brom oder Iod steht,

$R^4$
- für Wasserstoff, geradkettiges oder verzweigtes Alkyl mit bis zu 6 Kohlenstoffatomen oder für Phenyl steht

und

m
- für eine der Zahlen 2 oder 3 steht, und deren Salze.

**3.** 2-Halogensubstituierte N-Indolylethyl-sulfonamide der Formel I nach Anspruch 1
worin

$R^1$
- für Wasserstoff, Fluor, Chlor oder Trifluormethyl steht, oder
- für Methyl, Ethyl oder Propyl steht,
- für eine Gruppe der Formel $-NR^6R^7$ oder $-OR^8$ steht,

worin

$R^6$ und $R^7$ gleich oder verschieden sind und Wasserstoff, Methyl, Ethyl oder Propyl bedeuten,

$R^8$
- Wasserstoff, Methyl oder Ethyl bedeutet,

$R^2$
- für Methyl, Ethyl, Propyl, Isopropyl, Butyl oder tert.Butyl steht, das gegebenenfalls durch Fluor, Chlor, Phenyl, Hydroxy oder Cyano substituiert ist, oder
- für Phenyl steht, das gegebenenfalls bis zu 3-fach gleich oder verschieden durch Fluor, Chlor, Brom, Cyano, Trifluormethyl, Trifluormethoxy, Trifluormethylthio oder durch Methyl, Ethyl, Propyl, Isopropyl, Butyl, tert.Butyl, Methoxy, Ethoxy, Methoxycarbonyl, Ethoxycarbonyl, Methylthio, Ethylthio, Hydroxy, Carboxy, Phenyl, Phenoxy, Benzyloxy, Benzylthio oder durch eine Gruppe der Formel $-NR^6R^7$ substituiert ist,

worin
$R^6$ und $R^7$ die oben angegebene Bedeutung haben,

$R^3$
- für Fluor, Chlor oder Brom steht,

$R^4$
- für Wasserstoff, geradkettiges oder verzweigtes Alkyl mit bis zu 4 Kohlenstoffatomen oder Phenyl steht,

und

m
- für die Zahl 2 steht,

und deren Salze.

**4.** Verfahren zur Herstellung von Verbindungen der allgemeinen Formel

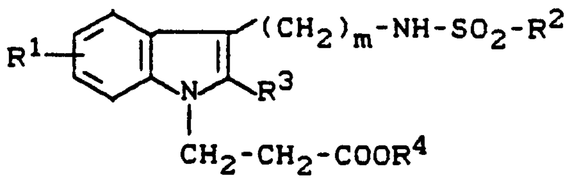

(I)

in welcher

$R^1$

- für Wasserstoff, Halogen, Trifluormethyl, Carboxy, Alkoxycarbonyl mit bis zu 8 Kohlenstoffatomen steht, oder
- für geradkettiges oder verzweigtes Alkyl mit bis zu 8 Kohlenstoffatomen oder für Cycloalkyl mit 3 bis 8 Kohlenstoffatomen steht, das gegebenenfalls durch Carboxy, Halogen, Alkoxy oder Alkoxycarbonyl mit jeweils bis zu 8 Kohlenstoffatomen, Cyano oder Hydroxy substituiert ist, oder
- für eine Gruppe der Formel $-S(O)_nR^5$, $-NR^6R^7$ oder $-OR^8$ steht,

worin

$R^5$

- geradkettiges oder verzweigtes Alkyl mit bis zu 8 Kohlenstoffatomen oder Aryl mit 6 bis 10 Kohlenstoffatomen bedeutet,

$R^6$ und $R^7$ gleich oder verschieden sind und Wasserstoff, geradkettiges oder verzweigtes Alkyl mit bis zu 8 Kohlenstoffatomen, Aryl mit 6 bis 10 Kohlenstoffatomen oder Acetyl bedeuten,

$R^8$

- Wasserstoff, geradkettiges oder verzweigtes Alkyl oder Alkylsulfonyl mit bis zu 8 Kohlenstoffatomen, Aryl oder Arylsulfonyl mit jeweils 6 bis 10 Kohlenstoffatomen oder Trifluormethyl bedeutet,

und

n

- eine Zahl 0, 1 oder 2 bedeutet

$R^2$

- für geradkettiges oder verzweigtes Alkyl mit bis zu 10 Kohlenstoffatomen steht, das gegebenenfalls durch Halogen, Phenyl, Hydroxy oder Cyano substituiert ist, oder
- für Aryl mit 6 bis 10 Kohlenstoffatomen steht, das gegebenenfalls bis zu 5-fach gleich oder verschieden durch Halogen, Cyano, Trifluormethyl, Trifluormethoxy, Trifluormethylthio oder durch Alkyl, Alkoxy, Alkoxycarbonyl oder Alkylthio mit jeweils bis zu 8 Kohlenstoffatomen, Hydroxy, Carboxy, Phenyl, Phenoxy, Benzyloxy, Benzylthio oder durch eine Gruppe der Formel $-NR^6R^7$ substituiert ist,

worin

$R^6$ und $R^7$ die oben angegebene Bedeutung haben,

$R^3$

- für Halogen steht,

$R^4$

- für Wasserstoff, geradkettiges oder verzweigtes Alkyl mit bis zu 8 Kohlenstoffatomen oder für Phenyl steht

und

m

- für eine der Zahlen 2, 3 oder 4 steht,

und deren Salze

dadurch gekennzeichnet, daß man Indole der allgemeinen Formel (II)

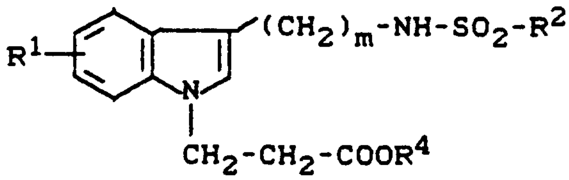

(II)

in welcher
$R^1$, $R^2$, $R^4$ und m die oben angegebene Bedeutung haben,
mit Halogen wie Fluor, Chlor oder Brom umsetzt,
und im Fall, daß $R^4$ für Alkyl oder Phenyl steht, mit Basen zu den entsprechenden Säuren ($R^4$ = Wasserstoff) verseift.

5. 2-Halogensubstituierte N-Indolylethyl-sulfonamide nach Anspruch 1 zur Bekämpfung von Krankheiten.

6. Arzneimittel enthaltend mindestens ein 2-halogensubstituiertes N-Indolylethylsulfonamid nach Anspruch 1.

7. Verfahren zur Herstellung eines Arzneimittels nach Anspruch 6, dadurch gekennzeichnet, daß man Verbindungen der Formel I nach Anspruch 1 gegebenenfalls mit Hilfe von üblichen Hilfs- und Trägerstoffen in eine geeignete Applikationsform bringt.

8. Verwendung der 2-halogensubstituierten N-Indolylethyl-sulfonamide nach Anspruch 1 zur Herstellung von Arzneimitteln.

9. Verwendung der 2-halogensubstiutierten N-Indolylethyl-sulfonamide nach Anspruch 1 zur Herstellung von Arzneimitteln zur Behandlung von Thrombosen, Thromboembolien, Ischämien, Asthma udn Allergien.

**Claims**

1. 2-Halogen-substituted N-indolylethyl-sulphonamides of the general formula (I)

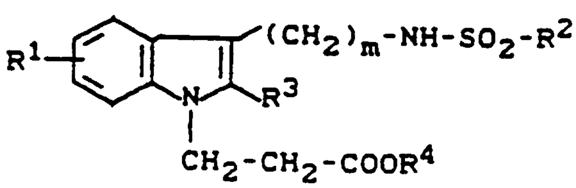

(I)

in which

$R^1$
- represents hydrogen, halogen, trifluoromethyl, carboxyl or alkoxycarbonyl having up to 8 carbon atoms, or
- represents straight-chain or branched alkyl having up to 8 carbon atoms, or represents cycloalkyl having 3 to 8 carbon atoms, which is optionally substituted by carboxyl, halogen, alkoxy or alkoxycarbonyl having in each case up to 8 carbon atoms, cyano or hydroxyl, or
- represents a group of the formula $-S(O)_nR^5$, $-NR^6R^7$ or $-OR^8$,

wherein

$R^5$
- denotes straight-chain or branched alkyl having up to 8 carbon atoms or aryl having 6 to 10 carbon atoms,

$R^6$ and $R^7$ are identical or different and denote hydrogen, straight-chain or branched alkyl having up to 8 carbon atoms, aryl having 6 to 10 carbon atoms or acetyl,

$R^8$
- denotes hydrogen, straight-chain or branched alkyl or alkylsulphonyl having up to 8 carbon atoms, aryl or arylsulphonyl having in each case 6 to 10 carbon atoms or trifluoromethyl,

and

n
- denotes the number 0, 1 or 2,

$R^2$
- represents straight-chain or branched alkyl having up to 10 carbon atoms, which is optionally substituted by halogen, phenyl, hydroxyl or cyano, or
- represents aryl having 6 to 10 carbon atoms, which is optionally substituted by up to 5 identical or different substituents from the group comprising halogen, cyano, trifluoromethyl, trifluoromethoxy, trifluoromethylthio, alkyl, alkoxy, alkoxycarbonyl or alkylthio having in each case up to 8 carbon atoms, hydroxyl, carboxyl, phenyl, phenoxy, benzyloxy and benzylthio, or by a group of the formula $-NR^6R^7$,

wherein
$R^6$ and $R^7$ have the abovementioned meaning,

$R^3$
- represents halogen,

$R^4$
- represents hydrogen or straight-chain or branched alkyl having up to 8 carbon atoms, or represents phenyl

and

m
- represents one of the numbers 2, 3 or 4,

and salts thereof.

**2.** 2-Halogen-substituted N-indolylethyl-sulphonamides of the formula (I) according to Claim 1, wherein

$R^1$
- represents hydrogen, fluorine, chlorine, bromine, trifluoromethyl, carboxyl or alkoxycarbonyl having up to 6 carbon atoms, or
- represents straight-chain or branched alkyl having up to 6 carbon atoms, or represents cycloalkyl having 3 to 6 carbon atoms, which is optionally substituted by carboxyl, fluorine, chlorine, bromine, alkoxy or alkoxycarbonyl having in each case up to 6 carbon atoms, cyano or hydroxyl, or
- represents a group of the formula $-S(O)_n-R^5$, $-NR^6R^7$ or $-OR^8$,

wherein

$R^5$
- denotes straight-chain or branched alkyl having up to 6 carbon atoms or phenyl,

$R^6$ and $R^7$ are identical or different and denote hydrogen, straight-chain or branched alkyl having up to 6 carbon atoms, phenyl or acetyl,

$R^8$
- denotes hydrogen, straight-chain or branched alkyl or alkylsulphonyl having in each case up to 6 carbon atoms, phenyl or trifluoromethyl,

and

n
- denotes the number 0, 1 or 2,

$R^2$
- represents straight-chain or branched alkyl having up to 8 carbon atoms, which is optionally substituted by fluorine, chlorine, bromine, phenyl, hydroxyl or cyano, or
- represents phenyl, which is optionally substituted by up to 3 identical or different substituents from the group comprising fluorine, chlorine, bromine, cyano, trifluoromethyl, trifluoromethoxy, trifluoromethylthio, alkyl, alkoxy, alkoxycarbonyl or alkylthio having in each case up to 6 carbon atoms, hydroxyl, carboxyl, phenyl, phenoxy, benzyloxy and benzylthio or by a group of the formula $-NR^6R^7$,

wherein
$R^6$ and $R^7$ have the abovementioned meaning,

$R^3$
- represents fluorine, chlorine, bromine or iodine,

$R^4$
- represents hydrogen or straight-chain or branched alkyl having up to 6 carbon atoms, or represents phenyl

and

m
- represents one of the numbers 2 or 3,

and salts thereof.

**3.** 2-Halogen-substituted N-indolylethyl-sulphonamides of the formula I according to Claim 1, wherein

$R^1$
- represents hydrogen, fluorine, chlorine or trifluoromethyl, or
- represents methyl, ethyl or propyl, or
- represents a group of the formula $-NR^6R^7$ or $-OR^8$,

wherein

$R^6$ and $R^7$ are identical or different and denote hydrogen, methyl, ethyl or propyl,

and

$R^8$
- denotes hydrogen, methyl or ethyl,

$R^2$
- represents methyl, ethyl, propyl, isopropyl, butyl or tert.-butyl, which is optionally substituted by fluorine, chlorine, phenyl, hydroxyl or cyano, or
- represents phenyl, which is optionally substituted by up to 3 identical or different substituents from the group comprising fluorine, chlorine, bromine, cyano, trifluoromethyl, trifluoromethoxy, trifluoromethylthio, methyl, ethyl, propyl, isopropyl, butyl, tert.-butyl, methoxy, ethoxy, methoxycarbonyl, ethoxycarbonyl, methylthio, ethylthio, hydroxyl, carboxyl, phenyl, phenoxy, benzyloxy and benzylthio, or by a group of the formula $-NR^6R^7$,

wherein

$R^6$ and $R^7$ have the abovementioned meaning,

$R^3$
- represents fluorine, chlorine or bromine,

$R^4$
- represents hydrogen, straight-chain or branched alkyl having up to 4 carbon atoms or phenyl

and

m
- represents the number 2,

and salts thereof.

**4.** Process for the preparation of compounds of the general formula

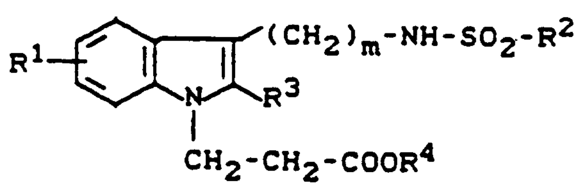

(I)

in which

$R^1$
- represents hydrogen, halogen, trifluoromethyl, carboxyl or alkoxycarbonyl having up to 8 carbon atoms, or

- represents straight-chain or branched alkyl having up to 8 carbon atoms, or represents cycloalkyl having 3 to 8 carbon atoms, which is optionally substituted by carboxyl, halogen, alkoxy or alkoxycarbonyl having in each case up to 8 carbon atoms, cyano or hydroxyl, or
- represents a group of the formula $-S(O)_nR^5$, $-NR^6R^7$ or $-OR^8$

wherein

$R^5$
- denotes straight-chain or branched alkyl having up to 8 carbon atoms or aryl having 6 to 10 carbon atoms,

$R^6$ and $R^7$ are identical or different and denote hydrogen, straight-chain or branched alkyl having up to 8 carbon atoms aryl having 6 to 10 carbon atoms or acetyl,

$R^8$
- denotes hydrogen, straight-chain or branched alkyl or alkylsulphonyl having up to 8 carbon atoms, aryl or arylsulphonyl having in each case 6 to 10 carbon atoms or trifluoromethyl,

and

n
- denotes the number 0, 1 or 2,

$R^2$
- represents straight-chain or branched alkyl having up to 10 carbon atoms, which is optionally substituted by halogen, phenyl, hydroxyl or cyano, or
- represents aryl having 6 to 10 carbon atoms, which is optionally substituted by up to 5 identical or different substituents from the group comprising halogen, cyano, trifluoromethyl, trifluoromethoxy, trifluoromethylthio, alkyl, alkoxy, alkoxycarbonyl and alkylthio having in each case up to 8 carbon atoms, hydroxyl, carboxyl, phenyl, phenoxy, benzyloxy and benzylthio, or by a group of the formula $-NR^6R^7$,

wherein

$R^6$ and $R^7$ have the abovementioned meaning,

$R^3$
- represents halogen,

$R^4$
- represents hydrogen or straight-chain or branched alkyl having up to 8 carbon atoms, or represents phenyl

and

m
- represents one of the numbers 2, 3 or 4,

and salts thereof,

characterized in that indoles of the general formula (II)

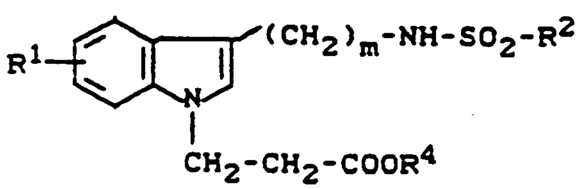 (II)

in which

$R^1$, $R^2$, $R^4$ and m have the abovementioned meaning,

are reacted with halogen, such as fluorine, chlorine or bromine, and, in the case where $R^4$ represents alkyl or phenyl, the products are hydrolysed with bases to give the corresponding acids ($R^4$ = hydrogen).

**5.** 2-Halogen-substituted N-indolylethyl-sulphonamides according to Claim 1 for combating diseases.

**6.** Medicaments containing at least one 2-halogen-substituted N-indolylethylsulphonamide according to Claim 1.

7. Process for the preparation of a medicament according to Claim 6, characterized in that compounds of the formula I according to Claim 1 are brought into a suitable administration form, if appropriate with the aid of customary auxiliaries and excipients.

8. Use of the 2-halogen-substituted N-indolylethylsulphonamides according to Claim 1 for the preparation of medicaments.

9. Use of the 2-halogen-substituted N-indolylethylsulphonamides according to Claim 1 for the preparation of medicaments for the treatment of thromboses, thromboembolisms, ischaemias, asthma and allergies.

**Revendications**

1. N-indolyléthyl-sulfonamides à substituant halogéné en position 2, de formule générale (I)

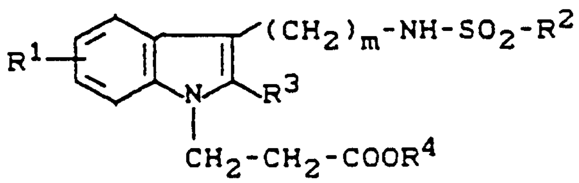

(I)

dans laquelle

$R^1$

- représente de l'hydrogène, un halogène, un groupe trifluorométhyle, carboxy, alkoxycarbonyle ayant jusqu'à 8 atomes de carbone, ou bien
- un groupe alkyle linéaire ou ramifié ayant jusqu'à 8 atomes de carbone ou un groupe cycloalkyle de 3 à 8 atomes de carbone, qui est substitué le cas échéant par un radical carboxy, halogéno, alkoxy ou alkoxycarbonyle ayant chacun jusqu'à 8 atomes de carbone, cyano ou hydroxy, ou bien
- un groupe de formule $-S(O)_nR^5$, $-NR^6R^7$ ou $-OR^8$,

où

$R^5$ désigne un radical alkyle linéaire ou ramifié ayant jusqu'à 8 atomes de carbone ou aryle ayant jusqu'à 10 atomes de carbone,

$R^6$ et $R^7$ sont identiques ou différents et représentent de l'hydrogène, un radical alkyle linéaire ou ramifié ayant jusqu'à 8 atomes de carbone, aryle de 6 à 10 atomes de carbone ou acétyle,

$R^8$ est de l'hydrogène, un radical alkyle ou alkylsulfonyle linéaire ou ramifié ayant jusqu'à 8 atomes de carbone, aryle ou arylsulfonyle ayant chacun 6 à 10 atomes de carbone, ou trifluorométhyle,

et

n représente le nombre 0, 1 ou 2

$R^2$

- est un groupe alkyle linéaire ou ramifié ayant jusqu'à 10 atomes de carbone, qui est éventuellement substitué par un halogène, un radical phényle, hydroxy ou cyano, ou bien
- un groupe aryle de 6 à 10 atomes de carbone, qui porte le cas échéant jusqu'à 5 substituants, identiques ou différents, halogéno, cyano, trifluorométhyle, trifluorométhoxy, trifluorométhylthio ou alkyle, alkoxy, alkoxycarbonyle ou alkylthio ayant chacun jusqu'à 8 atomes de carbone, hydroxy, carboxy, phényle, phénoxy, benzyloxy, benzylthio ou un groupe de formule $-NR^6R^7$,

dans laquelle

$R^6$ et $R^7$ ont la définition indiquée ci-dessus,

$R^3$

- est un halogène,

$R^4$

- est de l'hydrogène, un groupe alkyle linéaire ou ramifié ayant jusqu'à 8 atomes de carbone ou un groupe phényle

et

m

- représente l'un des nombres 2, 3 et 4, et leurs sels.

**2.** N-indolyléthylsulfonamides à substituant halogéno en position 2, de formule (I) suivant la revendication 1,

dans laquelle

$R^1$ représente de l'hydrogène, du fluor, du chlore, du brome, un groupe trifluorométhyle, carboxy, alkoxycarbonyle ayant jusqu'à 6 atomes de carbone, ou bien
un groupe alkyle linéaire ou ramifié ayant jusqu'à 6 atomes de carbone ou un groupe cycloalkyle de 3 à 6 atomes de carbone, qui porte le cas échéant un substituant carboxy, fluoro, chloro, bromo, alkoxy ou alkoxycarbonyle ayant chacun jusqu'à 6 atomes de carbone, cyano ou hydroxy, ou bien
un groupe de formule $-S(O)_n-R^5$, $-NR^6R^7$ ou $-OR^8$, où

$R^5$ est un groupe alkyle linéaire ou ramifié ayant jusqu'à 6 atomes de carbone ou un groupe phényle,

$R^6$ et $R^7$ sont identiques ou différents et représentent de l'hydrogène, un groupe alkyle linéaire ou ramifié ayant jusqu'à 6 atomes de carbone, phényle ou acétyle,

$R^8$ est de l'hydrogène, un groupe alkyle ou alkylsulfonyle linéaire ou ramifié ayant chacun jusqu'à 6 atomes de carbone, un groupe phényle ou trifluorométhyle,
et

n est le nombre 0, 1 ou 2,

$R^2$

- est un groupe alkyle linéaire ou ramifié ayant jusqu'à 8 atomes de carbone, qui est éventuellement substitué par du fluor, du chlore, du brome, un radical phényle, hydroxy ou cyano, ou bien
- un groupe phényle qui porte le cas échéant jusqu'à 3 substituants, identiques ou différents, fluoro, chloro, bromo, cyano, trifluorométhyle, trifluorométhoxy, trifluorométhylthio ou alkyle, alkoxy, alkoxycarbonyle ou alkylthio ayant chacun jusqu'à 6 atomes de carbone, hydroxy, carboxy, phényle, phénoxy, benzyloxy, benzylthio ou un groupe de formule $-NR^6R^7$,

dans laquelle
$R^6$ et $R^7$ ont la définition indiquée ci-dessus,

$R^3$ représente du fluor, du chlore, du brome ou de l'iode,

$R^4$ est de l'hydrogène, un groupe alkyle linéaire ou ramifié ayant jusqu'à 6 atomes de carbone ou un groupe phényle
et

m représente l'un des nombres 2 et 3, et leurs sels.

**3.** N-indolyléthylsulfonamides à substituant halogéno en position 2, de formule I suivant la revendication 1,

dans laquelle

$R^1$

- est de l'hydrogène, du fluor, du chlore ou un groupe trifluorométhyle, ou bien
- un groupe méthyle, éthyle ou propyle,
- un groupe de formule $-NR^6R^7$ ou $-OR^8$ dans laquelle

$R^6$ et $R^7$ sont identiques ou différents et représentent de l'hydrogène, un radical méthyle, éthyle ou propyle,

$R^8$

- est de l'hydrogène, un radical méthyle ou éthyle,

$R^2$

- est un groupe méthyle, éthyle, propyle, isopropyle, butyle ou tertiobutyle qui est substitué le cas échéant par du fluor, du chlore, un radical phényle, hydroxy ou cyano, ou bien
- un groupe phényle qui est substitué le cas échéant jusqu'à 3 fois, identiques ou différentes, par du fluor, du chlore, du brome, un radical cyano, trifluorométhyle, trifluorométhoxy, trifluorométhylthio ou par un radical méthyle, éthyle, propyle, isopropyle, butyle, tertiobutyle, méthoxy, éthoxy, méthoxycarbonyle, éthoxycarbonyle, méthylthio, éthylthio, hydroxy, carboxy, phényle, phénoxy, benzyloxy, benzylthio ou par un groupe de formule $-NR^6R^7$,
dans laquelle
$R^6$ et $R^7$ ont la définition indiquée ci-dessus,

$R^3$
- représente du fluor, du chlore ou du brome,

$R^4$
- est de l'hydrogène, un groupe alkyle linéaire ou ramifié ayant jusqu'à 4 atomes de carbone ou un radical phényle,

et

m
- représente le nombre 2,

et leurs sels.

**4.** Procédé de production de composés de formule générale

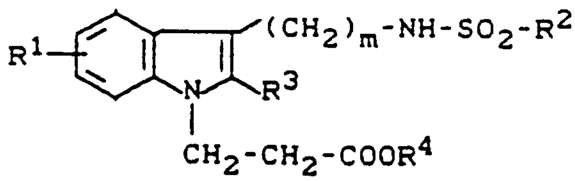

(I)

dans laquelle

$R^1$
- représente de l'hydrogène, un halogène, un groupe trifluorométhyle, carboxy, alkoxycarbonyle ayant jusqu'à 8 atomes de carbone, ou bien
- un groupe alkyle linéaire ou ramifié ayant jusqu'à 8 atomes de carbone ou un groupe cycloalkyle de 3 à 8 atomes de carbone, qui est substitué le cas échéant par un radical carboxy, halogéno, alkoxy ou alkoxycarbonyle ayant chacun jusqu'à 8 atomes de carbone, cyano ou hydroxy, ou bien
- un groupe de formule $-S(O)_nR^5$, $-NR^6R^7$ ou $-OR^8$,

où

$R^5$ désigne un radical alkyle linéaire ou ramifié ayant jusqu'à 8 atomes de carbone ou aryle ayant 6 à 10 atomes de carbone,

$R^6$ et $R^7$ sont identiques ou différents et représentent de l'hydrogène, un radical alkyle linéaire ou ramifié ayant jusqu'à 8 atomes de carbone, aryle de 6 à 10 atomes de carbone ou acétyle,

$R^8$ est de l'hydrogène, un radical alkyle ou alkylsulfonyle linéaire ou ramifié ayant jusqu'à 8 atomes de carbone, aryle ou arylsulfonyle ayant chacun 6 à 10 atomes de carbone, ou trifluorométhyle,

et

n représente le nombre 0, 1 ou 2

$R^2$
- est un groupe alkyle linéaire ou ramifié ayant jusqu'à 10 atomes de carbone, qui est éventuellement substitué par un halogène, un radical phényle, hydroxy ou cyano, ou bien
- un groupe aryle de 6 à 10 atomes de carbone, qui porte le cas échéant jusqu'à 5 substituants, identiques ou différents, halogéno, cyano, trifluorométhyle, trifluorométhoxy, trifluorométhylthio ou alkyle, alkoxy, alkoxycarbonyle ou alkylthio ayant chacun jusqu'à 8 atomes de carbone, hydroxy, carboxy, phényle, phénoxy, benzyloxy, benzylthio ou un groupe de formule $-NR^6R^7$,

dans laquelle
$R^6$ et $R^7$ ont la définition indiquée ci-dessus,

$R^3$
- est un halogène,

$R^4$

- est de l'hydrogène, un groupe alkyle linéaire ou ramifié ayant jusqu'à 8 atomes de carbone ou un groupe phényle

et

m

- représente l'un des nombres 2, 3 et 4 et de leurs sels,

caractérisé en ce qu'on fait réagir des indoles de formule générale (II)

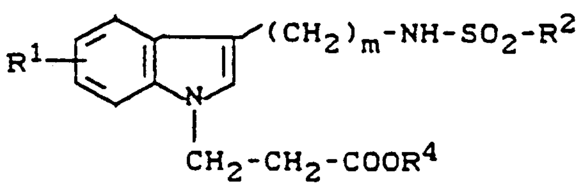

(II)

dans laquelle

$R^1$, $R^2$, $R^4$ et m ont la définition indiquée ci-dessus, avec un halogène tel que le fluor, le chlore ou le brome, et au cas où $R^4$ représente un groupe alkyle ou phényle, on effectue une saponification avec des bases en les acides correspondants ($R^4$ = hydrogène).

**5.** N-indolyléthylsulfonamides à substituant halogéno en position 2 suivant la revendication 1, destinés à combattre des maladies.

**6.** Médicament contenant au moins un N-indolyléthylsulfonamide portant un substituant halogéno en position 2 suivant la revendication 1.

**7.** Procédé de préparation d'un médicament suivant la revendication 6, caractérisé en ce qu'on met sous une forme administrable appropriée des composés de formule I suivant la revendication 1, le cas échéant à l'aide de substances auxiliaires et de supports classiques.

**8.** Utilisation des N-indolyléthylsulfonamides à substituant halogéno en position 2 suivant la revendication 1 pour la préparation de médicaments.

**9.** Utilisation des N-indolyléthylsulfonamides portant un substituant halogéno en position 2 suivant la revendication 1 pour la préparation de médicaments destinés au traitement de thromboses, de thromboembolies, d'ischémies, de l'asthme et d'allergies.